# EUROPEAN PATENT APPLICATION

(11) **EP 4 574 883 A1**
(43) Date of publication of application: **25.06.2025**
(21) Application number: 24222098.6
(22) Date of filing: 20.12.2024
(51) Int. Cl.: C08G 63/85

(54) **RECYCLED POLYBUTYLENE TEREPHTHALATE AND METHOD FOR MANUFACTURING THE SAME**

(30) Priority: 21.12.2023 TW 112149982
(71) Applicant: Shinkong Synthetic Fibers Corporation, Taipei City 106465 (TW)
(72) Inventor: TSENG, Yenhao, 106465 Taipei City (TW); HUANG, Pohao, 106465 Taipei City (TW); LIAO, Kunti, 106465 Taipei City (TW)
(74) Representative: Hoyng Rokh Monegier B.V.

(57) **Abstract**

The present invention discloses a recycled polybutylene terephthalate (PBT) and its production method thereof. The recycled polybutylene terephthalate is a polybutylene terephthalate obtained by chemically depolymerizing and ester exchanging recycled PET with 1,4 butanediol (BDO) directly using a Ti catalyst chelated by a polyacid or a polyalcohol, and then through polymerization. The production method is to reduce the manufacturing process and to produce a product with good physical properties.

## Description

### CROSS-REFERENCE TO RELATED APPLICATIONS

This application claims the benefit of priority of Taiwan Application No. 112149982, filed on December 21, 2023, and the disclosure of which is herein incorporated by reference in its entirety.

### FIELD OF THE INVENTION

The present invention relates to a method for recycling polyethylene terephthalate (PET) to produce polybutylene terephthalate (PBT) with good physical properties and the recycled polybutylene terephthalate produced by the mentioned method.

### BACKGROUND OF THE INVENTION

Waste plastics around the world are already an environmental problem that must be solved. In order to reduce the amount of polyethylene terephthalate (PET) waste, PET bottles and 1,4-butanediol (BDO) have been used as raw materials for preparation of polybutylene terephthalate (PBT) through chemical depolymerization, transesterification and polymerization, thereby achieving the recycling and reuse of polyethylene terephthalate. However, this method requires the use of an excessive amount of 1,4-butanediol, *i.e.,* the mole ratio of 1,4-butanediol to polyethylene terephthalate is greater than 3.2, which will lead to the increase of the production cost and the overconsumption of the raw materials, so it has not been commercialized.

The patent US7,799,836 applied by Sabic in 2007 claimed that the chemical depolymerization of polyethylene terephthalate by ethylene glycol (EG), followed by the addition of 1,4-butanediol to carry out the transesterification and polymerization, can effectively reduce the usage amount of 1,4-butanediol and achieve technology commercialization. However, the method disclosed in Sabic's patent US7,799,836 generates additionally a by-product diethylene glycol (DEG) during the depolymerization using ethylene glycol, which results in a lower melting point of the produced polybutylene terephthalate and affects its physical properties.

### SUMMARY OF THE INVENTION

In order to solve the above issues in the prior art and to achieve the purpose of the present invention, the present invention can directly use 1,4-butanediol for depolymerization and transesterification, followed by polymerization to obtain polybutylene terephthalate, and the usage amount of 1,4-butanediol can be greatly reduced, so that the mole ratio of 1,4-butanediol to polyethylene terephthalate can be reduced to 2.0. In addition, the direct use of 1,4-butanediol for depolymerization and transesterification has an advantage of reducing the number of steps in the process, as well as requiring lower temperature for alcoholysis with 1,4-butanediol compared to alcoholysis with ethylene glycol, which results in a more energy-efficient and economically valuable process.

Commonly used catalysts for depolymerization include manganese (Mn), zinc (Zn), antimony (Sb), titanium (Ti), etc., but manganese (Mn), zinc (Zn), antimony (Sb) catalysts are generally not used in polybutylene terephthalate materials. If the aforementioned catalysts are used, there will be side effects of yellowish color and poor heat resistance of polybutylene terephthalate. Therefore, we prefer to use titanium (Ti) catalyst as depolymerization catalyst. However, common titanium (Ti) catalyst, such as tetraisopropyl titanate (TiPT), tetra n-butyl titanate (TnBT) or the common commercial product tetrabutyl titanate (TBT), etc., will have the problem of the poor hue of the polyethylene terephthalate. In order to overcome the problem of the poor hue when titanium (Ti) catalyst is added to the depolymerization of the polyethylene terephthalate, titanium (Ti) catalyst with chelating a polybasic acid (e.g., a citric acid) or chelating a polyol (e.g., mannitol) is specially selected in the present invention. The use of the titanium (Ti) catalyst with chelating a polybasic acid or chelating a polyol as a depolymerization catalyst can avoid the side reaction of other metal catalysts on polybutylene terephthalate. In addition, such catalysts have selective capabilities and thus can improve the effects of the depolymerization of the polyethylene terephthalate and the transesterification of 1,4-butanediol, thereby reducing the usage amount of 1,4-butanediol. Furthermore, the recycled polybutylene terephthalate has good hue, as well as its physical properties is equivalent to those of brand new, non-recycled, polybutylene terephthalate.

One aspect of the present invention provides a method for manufacturing a recycled polybutylene terephthalate (recycled PBT), which has the following steps: (1). Under normal pressure, 220°C (or 210 to 230°C) and in the presence of a titanium catalyst, polyethylene terephthalate is mixed with 1,4-butanediol for depolymerization and transesterification to form a reaction mixture, wherein the titanium catalyst is chelated with a polybasic acid or a polyol, and the reaction mixture at least comprises tetrahydrofuran (THF), water, ethylene glycol and 1,4-butanediol; (2). After gradually vacuuming (reducing the pressure) to remove a part of the tetrahydrofuran and ethylene glycol from the reaction mixture, wherein at least 90% of the tetrahydrofuran and ethylene glycol can be removed by vacuum, the condensation polymerization is carried out at less than 0.1 atm and 245°C (235 to 255°C) to produce a recycled polybutylene terephthalate.

In an embodiment, the a polybasic acid for chelating the titanium catalyst comprise a citric acid or a tartaric acid, preferably a citric acid.

In an embodiment, the polyol for chelating the titanium catalyst comprise a xylitol, a sorbitol, a maltitol, an erythritol or a mannitol, preferably a mannitol.

In an embodiment, the polyethylene terephthalate is mixed with 1,4-butanediol for depolymerization and transesterification, and the mole ratio of the 1,4-butanediol to the polyethylene terephthalate is from 2:1 to 3.5:1, preferably from 2:1 to 3:1, and optimally from 2:1 to 2.5:1.

In an embodiment, the amount of the ethylene glycol is at least 30% by weight relative to the total amount of the tetrahydrofuran, water, ethylene glycol and 1,4-butanediol in the reaction mixture, which is formed in the step 1 of the preparation method of the present invention.

In an embodiment, the total amount of the tetrahydrofuran and water does not exceed 70% by weight relative to the total amount of the tetrahydrofuran, water, ethylene glycol and 1,4-butanediol in the reaction mixture, which is formed in the step 1 of the preparation method of the present invention.

Another aspect of the present invention provides a recycled polybutylene terephthalate produced by the aforementioned method. The recycled polybutylene terephthalate is a copolymer, which is formed by the polymerization of two or more monomers. The copolymer comprises a terephthalic acid unit, an isophthalic acid unit and a butanediol unit. In addition, the copolymer may further comprise an ethylene glycol unit, which are less than 0.1% by weight relative to the total amount of the recycled polybutylene terephthalate, but preferably the copolymer does not comprise any ethylene glycol unit.

In an embodiment, the recycled polybutylene terephthalate has a limiting viscosity (IV) of 0.5 to 1.0 dl/g, preferably 0.85 to 0.9 dl/g, and has a melting point of at least 215°C, preferably 216°C or higher, which is an excellent physical property close to the melting point (223°C) of the non-recycled, new polybutylene terephthalate material.

In an embodiment, the lightness (L) in the color model coordinates of CIE L*a*b* of the recycled polybutylene terephthalate is not less than 74.0, the b* value equal to or less than 4.0. That is, the recycled polybutylene terephthalate has a good hue.

In an embodiment, the recycled polybutylene terephthalate comprises a diethylene glycol unit, wherein the amount of the diethylene glycol unit is less than 0.3% by weight, preferably less than 0.27% by weight, more preferably less than 0.25% by weight, relative to the total amount of the recycled polybutylene terephthalate.

In an embodiment, the amount of the butanediol unit comprised in the recycled polybutylene terephthalate is more than 40.0% by weight relative to the total amount of the recycled polybutylene terephthalate.

Another aspect of the present invention provides a polyester fiber, which is produced from polyester pellets of the recycled polybutylene terephthalate of the present invention as raw materials through a melt spinning process, and which comprises the aforementioned recycled polybutylene terephthalate.

Another aspect of the present invention provides a polyester film, which comprises the aforementioned recycled polybutylene terephthalate.

### DETAILED DESCRIPTION OF THE INVENTION

Other aspects of the embodiments of the present invention will be described in more detail below. It should be understood that the present invention may be embodied in different forms and should not be construed as limited to the embodiments described in the present invention. In contrast, the embodiments of the present invention are provided for fuller and more complete disclosure of the present invention, and enable the person having ordinary skill in the art to understand and carry out the present invention.

Unless otherwise stated, any numerical values, such as a concentration or a concentration range described herein, are to be understood as being modified in all instances by the term "about." "About" means within an acceptable error range for the particular value as determined by one of ordinary skill in the art, which will depend in part on how the value is measured or determined, i.e., the limitations of the measurement system. Unless explicitly stated otherwise within the Examples or elsewhere in the Specification in the context of a particular assay, result or embodiment, "about" means within one standard deviation per the practice in the art, or a range of up to 5%, whichever is larger.

References to "one embodiment," "an embodiment," "some embodiments," etc., indicate that the embodiment described may include a particular feature, structure, aspect, or characteristic, but every embodiment may not necessarily include the particular feature, structure, aspect, or characteristic. Moreover, such phrases are not necessarily referring to the same embodiment. Further, when a particular feature, structure, aspect, or characteristic is described in connection with an embodiment, it is submitted that it is within the knowledge of one skilled in the art to effect such feature, structure, or characteristic in connection with other embodiments whether or not explicitly described.

All technical and scientific terms used in the specification and the appended claims, unless otherwise defined, are defined as those commonly known by a person with ordinary skills in the art. The singular forms "a," "an" and "the" include plural referents unless the context clearly dictates otherwise. In this application, the use of "or" or "and" means "and/or" unless stated otherwise. Furthermore, use of the term "including" as well as other forms, such as "include", "includes," and is not "included," limiting. The section headings used herein are for organizational purposes only and are not to be construed as limiting the subject matter described. Unless otherwise specified, all the material used herein is commercial and can be easily obtained.

The recycled polybutylene terephthalate herein refers to a copolymer formed by depolymerizing, transesterifying and polymerizing the polyethylene terephthalate obtained through recycling. The recycled polybutylene terephthalate, which is close to pure polybutylene terephthalate (pure PBT), has good hue and physical properties, and can be used in the manufacture of polyester fibers, polyester sheets or film materials.

### Method for Manufacturing the Recycled Polybutylene Terephthalate

One embodiment of the present invention provides a method for manufacturing the recycled polybutylene terephthalate, in which the polyethylene terephthalate obtained through recycling is used to produce the recycled polybutylene terephthalate in two-step process:
**Step 1**: At 1 atm, 210°C to 230°C (e.g., 220°C), and in the presence of a titanium catalyst with chelating a polybasic acid or chelating a polyol as a depolymerization catalyst, 1,4-butanediol is added and mixed with polyethylene terephthalate to carry out a depolymerization and transesterification to form a mixed solution. The mixed solution includes oligomers of polyethylene terephthalate (oligo-PET), oligomers of polyethylene isophthalate (oligo-PEI), oligomers of polybutylene terephthalate (oligo-PBT), oligomers of polybutylene isophthalate (oligo-PBI), ethylene glycol (EG), 1,4-butanediol (BDO), tetrahydrofuran (THF) and water. The above temperature may be 210°C, 211°C, 212°C, 213°C, 214°C, 215°C, 216°C, 217°C, 218°C, 219°C, 220°C, 221°C, 222°C, 223°C, 224°C, 225°C, 226°C, 227°C, 228°C, 229°C, 230°C or any value or range between any two of the aforementioned values. 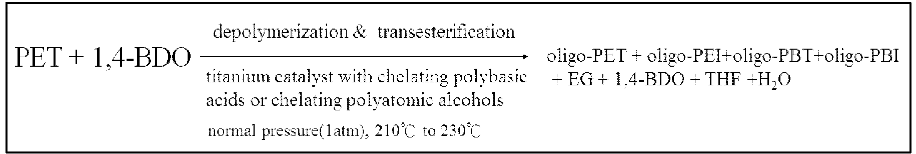
**Step 2**: Ethylene glycol and tetrahydrofuran in the mixed solution formed by the reaction in Step 1 are removed by gradually vacuuming. Under a pressure of less than 0.1 atm or near-vacuum, at a temperature of 235°C to 255°C, preferably 245°C, the condensation polymerization is carried out to form the recycled polybutylene terephthalate (r-PBT). In one embodiment, at least 90% of the ethylene glycol and tetrahydrofuran can be removed after vacuuming. The above temperature may be 235°C, 236°C, 237°C, 238°C, 239°C, 240°C, 241°C, 242°C, 243°C, 244°C, 245°C, 246°C, 247°C, 248°C, 249°C, 250°C, 251°C, 252°C, 253°C, 254°C, 255°C or any value or range between any two of the aforementioned values.

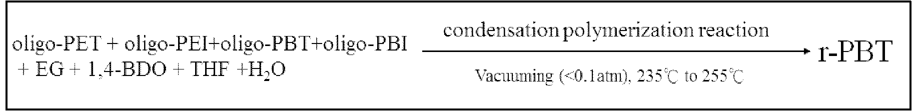

The term "depolymerization" in the present invention mainly refers to breaking the bonds of the ester group of the polyethylene terephthalate to degrade it into oligomers or diol and dibasic acid monomers, which can be recycled as raw materials for subsequent polymerization.

The term "transesterification" in the present invention mainly refers to replacing the ethylene glycol group of the initial ester with a butanediol group.

The term "condensation polymerization" refers to a chemical reaction in which two molecules are combined to form a new molecule by changes in functional groups, with some molecules being lost in the process. The formation of polyesters is a condensation polymerization, which is carried out by removing water molecules from carboxyl groups (-COOH) and hydroxyl group (-OH) and then forming ester groups.

In an embodiment, 1,4-butanediol is added and mixed with polyethylene terephthalate to carry out depolymerization and transesterification. The mole ratio of the 1,4-butanediol to the polyethylene terephthalate comprises from 2:1 to 3.5:1, for example, 2:1, 2.1:1, 2.2:1, 2.3:1, 2.4:1, 2.5:1, 2.6:1, 2.7. 1, 2.8:1, 2.9:1, 3.0:1, 3.1:1, 3.2:1, 3.3:1, 3.4:1, 3.5:1. In the present embodiment, the mole ratio of 1,4-butanediol to the polyethylene terephthalate is preferably 2:1 to 3:1, more preferably 2:1 to 2.5:1.

In an embodiment, in the reaction mixture of the aforementioned step 1, the amount of the ethylene glycol is at least 30% by weight relative to the total amount of the tetrahydrofuran, water, ethylene glycol and 1,4-butanediol, such as 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0% by weight or any value or range between any two of the aforementioned values. In addition, in the reaction mixture of the aforementioned step 1, the total amount of the tetrahydrofuran and water does not exceed 70% by weight relative to the total amount of the tetrahydrofuran, water, ethylene glycol and 1,4-butanediol, such as 70.0%, 69.5%, 69.0%, 68.5%, 68.0%, 67.5%, 67.0%, 66.5%, 66.0%, 65.5%, 65.0%, 64.5%, 64.0%, 63.5%, 63.0%, 62.5%, 62.0%, 61.5%, 61.0%, 60.5%, 60.0% by weight or any value or range between any two of the aforementioned values.

### Recycled Polybutylene Terephthalate

Another embodiment of the present invention is a recycled polybutylene terephthalate produced by the aforementioned method, which is a copolymer comprising a terephthalic acid unit, an isophthalic acid unit, and a butanediol unit. The physical properties of the recycled polybutylene terephthalate of the present embodiment can be specified by various parameters measured from the recycled polybutylene terephthalate, yarns and fabrics produced therefrom, as described below.
**(1). Intrinsic viscosity (IV)** is an intrinsic resistance of a polymer to flow, which can be measured by general technical methods in this field, such as the method according to ASTM D2857, in which the unit is usually represented by dl/g. For example, an intrinsic viscosity of polyester such as polyethylene terephthalate can be measured in a glass capillary viscometer using o-chlorophenol as solvent at 35°C at 1 g/dL polymer concentration. Alternatively, a thermoplastic polyester elastomer is dissolved in a solvent of phenol/tetrachloroethane (50/50 wt) to prepare a 0.5 % by weight solution and its viscosity is measured at 35°C using an Ubbelohde viscometer.
   According to the disclosure of the present application, the intrinsic viscosity is measured according to ASTM D2857. The intrinsic viscosity of the recycled polybutylene terephthalate in the present embodiment is between 0.5 to 1.0 dl/g, such as 0.50 dl/g, 0.55 dl/g, 0.60 dl/g, 0.65 dl/g , 0.70 dl/g, 0.75 dl/g, 0.80 dl/g, 0.81 dl/g, 0.82 dl/g, 0.83 dl/g, 0.84 dl/g, 0.85 dl/g, 0.86 dl/g, 0.87 dl/g , 0.88 dl/g, 0.89 dl/g, 0.90 dl/g, 0.91 dl/g, 0.92 dl/g, 0.93 dl/g, 0.94 dl/g, 0.95 dl/g, 0.96 dl/g, 0.97 dl/g , 0.98 dl/g, 0.99 dl/g, 1.00 dl/g or any value or range between any two of the aforementioned values.
**(2). Coordinates of CIE color model,** also written as L*a*b*, are expressed based on the coordinate diagram of color space formulated by the International Commission on Illumination (CIE). Among the three basic coordinates, L* represents the lightness of color, wherein the color is black when L* = 0 and it is white when L* = 100; a* represents the color between red and green, wherein the color is green when a* value is negative and it is red when a* value is positive; b* represents the color between yellow and blue, wherein the color is blue when b* value is negative and it is yellow when b* value is positive. Accordingly, the lightness will be low if the L* value is small, and the color will be yellowish if the b* value is large.
   The Lightness (L) in the coordinates of CIE color model of the recycled polybutylene terephthalate in the instant embodiment is not less than 74.0, such as 74.0, 74.5, 75.0, 75.5, 76.0, 76.5, 77.0, 77.5, 78.0, 78.5, 79.0, 79.5, 80.0 or any value or range between any two of the aforementioned values. Moreover, the b* value thereof is not greater than 4.0, such as 4.0, 3.5, 3.0, 2.5, 2.0, 1.5, 1.0 or any value or range between any two of the aforementioned values.
**(3). Melting point** is obtained by using Differential Scanning Calorimetry (DSC) to measure the object in accordance with the measurement method of ISO11357-1. The basic principle thereof is that when a sample undergoes phase change, glass transition and chemical reactions, the sample will absorb or release heat, and then the compensator can measure how to increase or decrease the heat flow to keep the sample and reference at the same temperature. The melting point of the recycled polybutylene terephthalate in the instant embodiment is at least 215°C, such as 215°C, 215.5°C, 216°C, 216.5°C, 217°C, 217.5°C, 218°C, 218.5°C, 219°C, 219.5°C, 220°C, 220.5°C, 221°C, 221.5°C, 222°C, 222.5°C, 222.3°C or any value or range between any two of the aforementioned values.

In addition, the recycled polybutylene terephthalate in the instant embodiment can further comprise an ethylene glycol unit, wherein the amount of the ethylene glycol unit is less than 0.1% by weight relative to the total amount of the recycled polybutylene terephthalate, such as 0.099%, 0.095%, 0.09%, 0.08%, 0.07%, 0.06%, 0.05%, 0.04%, 0.03%, 0.02%, 0.01%, 0.005%, 0.001% by weight or any value or range between any two of the aforementioned values.

In an embodiment, the recycled polybutylene terephthalate in the instant embodiment substantially does not comprise any ethylene glycol unit.

In an embodiment, the amount of the butanediol unit is more than 40.0% by weight relative to the total amount of the recycled polybutylene terephthalate, such as 40.05%, 40.1%, 40.2%, 40.3%, 40.4%, 40.5%, 40.6%, 40.7%, 40.8%, 40.9%, 41.0%, 41.1%, 41.2%, 41.3%, 41.4%, 41.5%, 42.0% by weight or any value or range between any two of the aforementioned values.

### Polyester Fiber

The disclosure of the present application provides another embodiment, which is a polyester fiber comprising the recycled polybutylene terephthalate of the aforementioned embodiments or the recycled polybutylene terephthalate produced by the method of the aforementioned embodiment. The recycled polybutylene terephthalate of the aforementioned embodiments can be heated and melted by a screw extruder, then quantitatively extruded through a spinning nozzle at a spinning temperature of 160 to 300°C, and then cooled and solidified by cooling air and oiled, followed by being spun and stretched to obtain polyester fibers.

### Polyester Film

The disclosure of the present application provides another embodiment, which is a polyester film comprising the recycled polybutylene terephthalate of the aforementioned embodiments. The recycled polybutylene terephthalate of the aforementioned embodiments can be melted and extruded at a temperature of 230 to 300°C to produce a polyester sheet, which is further processed by stretching to produce a polyester film.

Except for the above recitation of the embodiments, the remaining details of the embodiments of the present disclosure can be implemented with reference to the general skills in the art. Some specific examples are listed below for further explanation. However, the claimed invention is not limited to the specific examples listed below. The specific examples listed in the present disclosure are for illustrative purposes only. The claimed invention of the present disclosure may be extended to similar products with the same concept. While any methods and materials similar or equivalent to those described herein may be used in carrying out or experimenting with the embodiments and/or examples of the claimed invention, the disclosure herein is to the preferred methods and materials. All documents referred to in the present disclosure are incorporated herein by reference in their entirely.

### Example 1

1,4-butanediol and polyethylene terephthalate were put into the reactor at a mole ratio of 3.2:1 and mixed with an appropriate amount of titanium catalyst with chelating a citric acid at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate, *i.e.* polyester pellets of **recycled PBT.** The melting point (Tm) of the polyester pellets of the recycled polybutylene terephthalate is 216±1°C.

### Example 2

1,4-butanediol and polyethylene terephthalate were put into the reactor at a mole ratio of 2.5:1 and mixed with an appropriate amount of titanium catalyst with chelating a citric acid at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 216±1°C.

### Comparative Example 1

1,4-butanediol and polyethylene terephthalate were put into the reactor at a mole ratio of 3.2:1 and mixed without any catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 214±1°C.

### Comparative Example 2

1,4-butanediol and polyethylene terephthalate were put into the reactor at a mole ratio of 3.2:1 and mixed with an appropriate amount of a zinc (Zn) catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 214±1°C.

### Comparative Example 3

1,4-butanediol and polyethylene terephthalate were put into the reactor at a mole ratio of 3.2:1 and mixed with an appropriate amount of a manganese (Mn) catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 214±1°C.

### Comparative Example 4

1,4-butanediol and polyethylene terephthalate were put into the reactor at a mole ratio of 3.2:1 and mixed with an appropriate amount of antimony (Sb) catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 214±1°C.

### Comparative Example 5

1,4-butanediol and polyethylene terephthalate were put into the reactor at a mole ratio of 3.2:1 and mixed with an appropriate amount of titanium (Ti) catalyst without chelating ligands at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 216±1°C.

### Comparative Example 6

1,4-butanediol and polyethylene terephthalate were put into the reactor at a mole ratio of 2.5:1 and mixed without any catalyst 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 210±1°C.

### Comparative Example 7

1,4-butanediol and polyethylene terephthalate were put into the reactor at a mole ratio of 2.5:1 and mixed with an appropriate amount of a zinc (Zn) catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 212±1°C.

### Comparative Example 8

1,4-butanediol and polyethylene terephthalate were put into the reactor at a mole ratio of 2.5:1 and mixed with an appropriate amount of a manganese (Mn) catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 212±1°C.

### Comparative Example 9

1,4-butanediol and polyethylene terephthalate were put into the reactor at a mole ratio of 2.5:1 and mixed with an appropriate amount of antimony (Sb) catalyst at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 210±1°C.

### Comparative Example 10

1,4-butanediol and polyethylene terephthalate were put into the reactor at a mole ratio of 2.5:1 and mixed with an appropriate amount of titanium (Ti) catalyst without chelating ligands at 1 atm, 220°C, to carry out depolymerization and transesterification. After the depolymerization and transesterification were completed, the reactor was gradually vacuumed to remove ethylene glycol and tetrahydrofuran and then a condensation polymerization was carried out under a pressure of less than 0.1 atm and at 245°C. When the viscosity during the condensation polymerization reached the target viscosity (IV=0.88±0.02 dl/g), the cooled products were taken out from the reactor to be granulated to produce polyester pellets of the recycled polybutylene terephthalate. The melting point of the polyester pellets of the recycled polybutylene terephthalate is 214±1°C.

The properties of the aforementioned polyester pellets of the recycled polybutylene terephthalate produced with different catalysts or different ratios of 1,4-butanediol are summarized in Table 1:

**Table 1: Comparison of the properties of the polyester pellets of the recycled polybutylene terephthalate by different preparation processes**

| | a mole ratio (MR) of BDO:PET | depolymerization catalyst | IV (dl/g) | Color | | | Tm |
|---|---|---|---|---|---|---|---|
| | | | | L | a | b | |
| Example 1 | 3.2 : 1 | Ti with chelating ligands | 0.87±0.01 | 75.0±1.0 | -1.5±0.5 | +3.0±1.0 | 216±1°C |
| Example 2 | 2.5 : 1 | Ti with chelating ligands | 0.87±0.01 | 75.0±1.0 | -1.5±0.5 | +3.0±1.0 | 216±1°C |
| Comparative Example 1 | 3.2 : 1 | not added | 0.87±0.01 | 73.0±1.0 | -1.5±0.5 | +10.0±1.0 | 214±1°C |
| Comparative Example 2 | 3.2 : 1 | Zn | 0.87±0.01 | 73.0±1.0 | -1.5±0.5 | +7.0±1.0 | 214±1°C |
| Comparative Example 3 | 3.2 : 1 | Mn | 0.87±0.01 | 73.0±1.0 | -1.5±0.5 | +7.0±1.0 | 214±1°C |
| Comparative Example 4 | 3.2 : 1 | Sb | 0.87±0.01 | 70.0±1.0 | -1.5±0.5 | +10.0±1.0 | 214±1°C |
| Comparative Example 5 | 3.2 : 1 | Ti | 0.87±0.01 | 75.0±1.0 | -1.5±0.5 | +5.0±1.0 | 216±1°C |
| Comparative Example 6 | 2.5 : 1 | not added | 0.87±0.01 | 65.0±1.0 | -1.5±0.5 | +10.0±1.0 | 210±1°C |
| Comparative Example 7 | 2.5 : 1 | Zn | 0.87±0.01 | 70.0±1.0 | -1.5±0.5 | +7.0±1.0 | 212±1°C |
| Comparative Example 8 | 2.5 : 1 | Mn | 0.87±0.01 | 70.0±1.0 | -1.5±0.5 | +7.0±1.0 | 212±1°C |
| Comparative Example 9 | 2.5 : 1 | Sb | 0.87±0.01 | 63.0±1.0 | -1.5±0.5 | +10.0±1.0 | 210±1°C |
| Comparative Example 10 | 2.5 : 1 | Ti | 0.87±0.01 | 73.0±1.0 | -1.5±0.5 | +5.0±1.0 | 214±1°C |

As can be seen from the results in Table 1, when the mole ratio of 1,4-butanediol to polyethylene terephthalate is high, such as the mole ratio of 3.2 in Example 1 and Comparative Examples 1 to 5, the melting point of the obtained recycled polybutylene terephthalate would be close to that of pure polybutylene terephthalate regardless of whether a catalyst is added or what type of catalyst is added. In addition, when zinc (Zn), manganese (Mn), antimony (Sb) and other uncommon catalysts of polybutylene terephthalate are added, it will affect the hue of the recycled polybutylene terephthalate after polymerization, so that its lightness (L) decreases to be less than 75 and it is darker, or the b* value increases to be greater than +5.0 or even as high as +10.0 and it is yellowish.

When the mole ratio of 1,4-butanediol to polyethylene terephthalate decreases, such as the mole ratio of 2.5 in Example 2 and Comparative Examples 6 to 10, the melting point of the obtained recycled polybutylene terephthalate would drop significantly, except for Example 2 using titanium catalyst with chelating ligands. Therefore, only when titanium catalyst with chelating ligands is used as a catalyst for the depolymerization of polyethylene terephthalate and 1,4-butanediol, the melting point and hue of the obtained recycled polybutylene terephthalate could be maintained and not be affected.

The depolymerization time and polymerization time for each of the above examples and comparative examples are summarized in Table 2:

**Table 2: Comparison of the depolymerization time and polymerization time for different preparation processes**

| | a mole ratio (MR) of BDO:PET | depolymerization catalyst | depolymerization time | polymerization time |
|---|---|---|---|---|
| Example 1 | 3.2 : 1 | Ti with chelating ligands | 2h30m | 2h00m |
| Example 2 | 2.5 : 1 | Ti with chelating ligands | 2h30m | 2h00m |
| Comparative Example 1 | 3.2 : 1 | not added | 4h00m | 2h30m |
| Comparative Example 2 | 3.2 : 1 | Zn | 3h30m | 2h30m |
| Comparative Example 3 | 3.2 : 1 | Mn | 3h30m | 2h30m |
| Comparative Example 4 | 3.2 : 1 | Sb | 4h00m | 2h30m |
| Comparative Example 5 | 3.2 : 1 | Ti | 3h00m | 2h00m |
| Comparative Example 6 | 2.5 : 1 | not added | 4h00m | 2h30m |
| Comparative Example 7 | 2.5 : 1 | Zn | 3h30m | 2h30m |
| Comparative Example 8 | 2.5 : 1 | Mn | 3h30m | 2h30m |
| Comparative Example 9 | 2.5 : 1 | Sb | 4h00m | 2h30m |
| Comparative Example 10 | 2.5 : 1 | Ti | 3h00m | 2h00m |

As can be seen from the results in Table 2, the efficiencies of depolymerization and transesterification are different under different catalysts, resulting in difference in reaction time. Among them, the titanium (Ti) catalysts with chelating ligands used in Examples 1 and 2 as depolymerization catalysts take the shortest time in the depolymerization, which is 2 hours and 30 minutes, and their time for the subsequent polymerization is 2 hours. In comparison, the depolymerization time using zinc (Zn), manganese (Mn) or antimony (Sb) catalysts is about 3 hours 30 minutes to 4 hours, and their polymerization time is 2 hours 30 minutes, both of which are longer than the depolymerization time and the polymerization time using titanium (Ti) catalyst with chelating ligands as a depolymerization catalyst. Therefore, the use of titanium catalyst with chelating ligands as a depolymerization catalyst can significantly shorten the overall process time. In addition, by comparing Examples 1 and 2 with Comparative Examples 5 and 10, the depolymerization time (2 hours 30 minutes) using titanium catalyst with chelating ligands as a depolymerization catalyst is 30 minutes shorter than the depolymerization time (3 hours) using titanium catalyst without chelating ligands. It is also effective in shortening the overall process time.

The polyester pellets of recycled polybutylene terephthalate produced in the aforementioned Examples and Comparative Examples comprise a terephthalic acid unit, an isophthalic acid (IPA) unit, an ethylene glycol unit, a diethylene glycol unit and a 1,4-butanediol unit, and their ratios % by weight are summarized in Table 3:

**Table 3: Compositional analysis of the polyester pellets of recycled polybutylene terephthalate**

| | a mole ratio (MR) of BDO:PET | depolymerization catalyst | **GC (wt%)** | | | | Tm |
|---|---|---|---|---|---|---|---|
| | | | **IPA** | **EG** | **DEG** | **BDO** | |
| Example 1 | 3.2 : 1 | Ti with chelating ligands | 1.05 | **ND** | 0.25 | 40.53 | 216±1°C |
| Example 2 | 2.5 : 1 | Ti with chelating ligands | 1.03 | **0.08** | 0.27 | 40.41 | 216±1°C |
| Comparative Example 1 | 3.2 : 1 | not added | 1.06 | 0.61 | 0.44 | 38.90 | 214±1°C |
| Comparative Example 2 | 3.2 : 1 | Zn | 1.03 | 0.67 | 0.39 | 39.05 | 214±1°C |
| Comparative Example 3 | 3.2 : 1 | Mn | 0.99 | 0.63 | 0.43 | 39.11 | 214±1°C |
| Comparative Example 4 | 3.2 : 1 | Sb | 1.11 | 0.60 | 0.40 | 38.96 | 214±1°C |
| Comparative Example 5 | 3.2 : 1 | Ti | 1.01 | 0.10 | 0.27 | 40.30 | 216±1°C |
| Comparative Example 6 | 2.5 : 1 | not added | 1.10 | 2.12 | 1.28 | 36.14 | 210±1°C |
| Comparative Example 7 | 2.5 : 1 | Zn | 1.02 | 1.11 | 0.68 | 37.85 | 212±1°C |
| Comparative Example 8 | 2.5 : 1 | Mn | 0.98 | 1.14 | 0.73 | 37.72 | 212±1°C |
| Comparative Example 9 | 2.5 : 1 | Sb | 1.05 | 2.16 | 1.30 | 36.10 | 210±1°C |
| Comparative Example 10 | 2.5 : 1 | Ti | 1.06 | 0.55 | 0.46 | 39.20 | 214±1°C |

As can be seen from the results in Table 3, when the mole ratio of 1,4-butanediol to polyethylene terephthalate is 3.2, the ratios of components in the polyester pellets of recycled polybutylene terephthalate obtained by using different depolymerization catalysts, such as zinc (Zn), manganese (Mn) or antimony (Sb) catalyst, are similar, wherein the ratio of IPA is in the range of 1.03% to 1.11% by weight, the ratio of EG is in the range of 0.60% to 0.67% by weight, the ratio of DEG is in the range of 0.39% to 0.43% by weight and the ratio of BDO is in the range of 38.96% to 39.11% by weight. Therefore, these polyester pellets also have similar melting points of 214±1°C. In addition, the polyester pellets of recycled polybutylene terephthalate obtained by using titanium (Ti) catalyst with chelating ligands or titanium catalyst without chelating ligands have similar compositions, wherein the ratio of IPA is in the range of 1.01% to 1.05% by weight, the ratio of EG is in the range of 0% to 0.10% by weight, the ratio of DEG is in the range of 0.25% to 0.27% by weight and the ratio of BDO is in the range of 40.30% to 40.53% by weight, and thus also have similar melting points.

However, when the amount of 1,4-butanediol is reduced (e.g., mole ratio of 2.5), the residual amounts of ethylene glycol and diethylene glycol increase significantly in the polyester pellets of recycled polybutylene terephthalate obtained by using zinc, manganese, antimony catalyst or titanium catalyst without chelating ligands. For example, the residual amount of ethylene glycol increases to 0.55% to 2.16% by weight and that of diethylene glycol also increases to 0.46% to 1.30% by weight, resulting in a significant decrease in the melting points of these polyester pellets. When the residual amount of ethylene glycol is 0.55% by weight and that of diethylene glycol is 0.46% by weight, the melting point of the polyester pellets decreases to 214±1°C; when the residual amount of ethylene glycol increases to 1.11% to 1.14% by weight and that of diethylene glycol increases to 0.68% to 0.73% by weight, the melting point of the polyester pellets decreases to 212±1°C; when the residual amount of ethylene glycol increases to 2.16% by weight and that of diethylene glycol increases to 1.30% by weight, the melting point of the polyester pellets further decreases to 210±1°C. The results in Table 3 also show that, when the titanium catalyst with chelating ligands is used as a depolymerization catalyst, the polyester pellets of recycled polybutylene terephthalate are almost free of ethylene glycol (as in Example 2), or even no ethylene glycol is detected at all in the polyester pellets (as in Example 1), and the residual amount of diethylene glycol as by-product is less than 0.27% by weight, which is the lowest amount of diethylene glycol among all Examples and Comparative Examples. In other words, when titanium catalyst with chelating a polybasic acid or a polyol is used as a depolymerization catalyst, it is possible to maintain low level of residual ethylene glycol and diethylene glycol in the recycled polybutylene terephthalate without using so much 1,4-butanediol, resulting in the melting point thereof is equivalent to that of the recycled polybutylene terephthalate produced with a high dosage of 1,4-butanediol.

Among the aforementioned Examples and Comparative Examples, the distillates of the reaction mixtures, which are produced from polyethylene terephthalate and 1,4-butanediol by the depolymerization and transesterification of Step 1, are analyzed to contain tetrahydrofuran, water, ethylene glycol, and 1,4-butanediol. Their ratios % by weight are summarized in Table 4:

**Table 4: Compositional analysis of the distillate of recycled polybutylene terephthalate**

| | depolymerization catalyst | **GC (wt%)** | | | |
|---|---|---|---|---|---|
| | | **IPA** | **EG** | **DEG** | **BDO** |
| Comparative Example 6 | not added | 50.22 | 14.05 | **17.90** | 15.33 |
| Comparative Example 7 | Zn | **57.08** | 18.02 | 21.14 | 2.01 |
| Comparative Example 8 | Mn | **56.78** | 17.83 | 20.57 | 3.09 |
| Comparative Example 9 | Sb | 50.61 | 15.00 | **16.56** | 16.10 |
| Comparative Example 10 | Ti | 52.01 | 16.01 | 28.01 | 2.03 |
| Example 2 | **Ti with chelating ligands** | **50.13** | **13.61** | **31.50** | **1.92** |

The reaction mixtures after depolymerization and transesterification comprise not only components with higher boiling points, such as oligomers of polyethylene terephthalate, oligomers of polyethylene isophthalate, oligomers of polybutylene terephthalate and oligomers of polybutylene isophthalate, but also components with low boiling points, such as tetrahydrofuran, water, ethylene glycol and 1,4-butanediol. The components with low boiling points of the reaction mixtures are present in the distillates of the reaction mixtures, as evidenced by the results of the compositional analysis of the distillate in Table 4. Due to the substitution or conversion relationship among the four main compounds, i.e., tetrahydrofuran, water, ethylene glycol and 1,4-butanediol, consisting of the distillate (for example, an increase in the amount of distilled ethylene glycol usually represents a successful substitution of 1,4-butanediol for a ethylene glycol unit in the depolymerized oligomers and consequent decrease in the amount of 1,4-butanediol in the distillate; in addition, 1,4-butanediol may also be dehydrated to form tetrahydrofuran, resulting in a decrease in the amount of 1,4-butanediol and an increase in tetrahydrofuran and water) and the substitution or conversion relationship will directly affect the composition of the final product. Therefore, the relative difference among the ratios of tetrahydrofuran, water, ethylene glycol and 1,4-butanediol can be used to simply and clearly determine the impact of different depolymerization catalysts on depolymerization and transesterification.

As can be seen from the analysis results of distillate composition in Table 4, the depolymerization and transesterification of 1,4-butanediol without the addition of any catalyst or with the addition of antimony (Sb) catalyst are not very effective, and it cannot be effective to separate ethylene glycol from the polyethylene terephthalate and distill out the ethylene glycol. Therefore, the mixture produced by depolymerization and transesterification with antimony (Sb) catalyst comprise has a significantly lower ratio % of ethylene glycol by weight (less than 20% by weight) in its distillate. In Example 2, the ethylene glycol accounts for 31.50% by weight of the distillate after depolymerization and transesterification by adding titanium catalyst with chelating ligands. In comparison with the results of Comparative Example 6 without adding any catalyst and Comparative Example 10 of adding antimony catalyst, the ethylene glycol only accounts for 16.56% and 17.90% by weight of the distillate and the ratio of ethylene glycol significantly reduces by about 13 to 15% by weight. In addition, while the addition of zinc (Zn) or manganese (Mn) catalyst can improve the depolymerization and transesterification ability of 1,4-butanediol, resulting in slightly more than 20% by weight of ethylene glycol being substituted and distilled out of the polyethylene terephthalate, zinc (Zn) or manganese (Mn) catalyst has the side effect of contributing to the dehydration of 1,4-butanediol and the formation of tetrahydrofuran, which will result in insufficient free 1,4-butanediol to completely substitute ethylene glycol during the polymerization, wherein the free 1,4-butanediol can participate in the polymerization. In Example 2, in which titanium catalyst with chelating ligands was added, the total amount of tetrahydrofuran and water accounts for 63.74% by weight of the distillate. However, when the depolymerization catalyst was replaced with zinc or manganese catalyst in Comparative Example 7 and Comparative Example 8, the total amount of tetrahydrofuran and water accounts for 75.10% and 74.61% by weight of the distillate, which increases by 11.36% and 10.87% by weight in comparison with the total amount of tetrahydrofuran and water in Example 2.

As can be seen from the analysis results of distillate composition in Table 4, according to the residual amount of 1,4-butanediol (BDO), the use of titanium catalyst without chelating ligands in Comparative Example 10 (the residual amount of 1,4-butanediol of 2.03% by weight) is more effective in the depolymerization and transesterification of 1,4-butanediol in comparison with no use of catalyst in Comparative Example 6 (the residual amount of 1,4-butanediol of 15.33% by weight) and the use of antimony catalyst in Comparative Example 9 (the residual amount of 1,4-butanediol of 16.10% by weight). Moreover, according to the residual total amount of tetrahydrofuran and water, the use of titanium catalyst without chelating ligands in Comparative Example 10 (the residual total amount of tetrahydrofuran and water of 68.02% by weight) has less impact on the formation of the by-product tetrahydrofuran in comparison with the use of zinc (Zn) catalyst in Comparative Example 7 (the residual total amount of tetrahydrofuran and water of 75.10% by weight) and the use of manganese (Mn) catalyst in Comparative Example 8 (the residual total amount of tetrahydrofuran and water of 74.61% by weight). However, the reaction between polyethylene terephthalate and 1,4-butanediol with chelating ligands has the best effect on depolymerization and transesterification and is least affected by the formation of the by-product tetrahydrofuran. In Example 2, in which titanium catalyst with chelating ligands was used, the amount of distilled ethylene glycol accounts for more than 30% by weight relative to the amount of the distillate or the total amount of main components, i.e., tetrahydrofuran, water, ethylene glycol and 1,4-butanediol, of the distillate. In comparison, the results in which titanium catalyst without chelating ligands or catalyst other than titanium catalyst with chelating ligands was used, show that the amount of distilled ethylene glycol accounts for less than 30% or even only 16% (in Comparative Example 9, in which antimony catalyst was used) by weight of the amount of the distillate or the total amount of main components of the distillate. The results show that the use of titanium catalyst with chelating ligands has significant effects on depolymerization and transesterification and can also effectively reduce the proportion of an ethylene glycol unit in the recycled polybutylene terephthalate after subsequent polymerization. In Example 2, the total amount of the tetrahydrofuran and water accounts for only about 65% and less than 70% by weight of the amount of the distillate or the total amount of main components of the distillate. The proportion of tetrahydrofuran and water in Example 2 is significantly lower than that (about 75% by weight) in which zinc or manganese catalyst is used and also lower than that (about 68 to 69% by weight) in which titanium catalyst without chelating ligands is used. These results also show that the formation of by-product tetrahydrofuran is effectively reduced.

Moreover, the ratios of 1,4-butanediol, ethylene glycol, tetrahydrofuran and water produced after the depolymerization of polyethylene terephthalate and 1,4-butanediol with different catalysts are summarized in Table 5:

**Table 5: Analysis of components participating in the polymerization of recycled polybutylene terephthalate:**

| depolymerization catalyst | **GC (wt%)** | | | |
|---|---|---|---|---|
| | **THF** | **H2O** | **EG** | **BDO** |
| not added | 1.35 | 4.01 | 11.33 | 82.40 |
| Zn | 0.92 | 3.10 | 8.01 | 86.01 |
| Mn | 0.88 | 3.01 | 7.83 | 86.77 |
| Sb | 1.41 | 3.88 | 10.77 | 83.01 |
| Ti | 0.57 | 2.84 | 5.19 | 89.41 |
| **Ti with chelating ligands** | 0.33 | 2.01 | 4.19 | **92.01** |

As can be seen from the analysis results in Table 5, after the depolymerization with titanium catalyst with chelating ligands, the maximum amount of free 1,4-butanediol can participate in the polymerization, so that the proportion of butanediol unit in the recycled polybutylene terephthalate increases and the recycled polybutylene terephthalate with the best melting point can be produced.

Referring also to Table 3 for the results of compositional analysis of the polyester pellets of recycled polybutylene terephthalate, the ratios % of various components (an isophthalic acid unit, an ethylene glycol unit, a diethylene glycol unit, a 1,4-butanediol unit and a terephthalic acid unit) by weight are converted into that by mole (mole percentage). In further analysis, when the mole ratio of 1,4-butanediol to polyethylene terephthalate is 2.5, the ratios% of total amount of ethylene glycol unit and a diethylene glycol unit and the amount of 1,4-butanediol unit by mole in the said polyester pellets are summarized in Table 6:

**Table 6: analysis of the ratios% of EG, DEG and BDO by mole in the polyester pellets of recycled polybutylene terephthalate**

| | a mole ratio (MR) of BDO:PET | depolymerization catalyst | **GC (wt%)** | | | |
|---|---|---|---|---|---|---|
| | | | **EG** | **DEG** | **EG+DEG** | **BDO** |
| **Example 2** | **2.5 : 1** | **Ti with chelating ligands** | **0.16** | **0.31** | **0.47** | **55.44** |
| Comparative Example 6 | 2.5 : 1 | not added | 4.21 | 1.49 | 5.70 | 49.44 |
| Comparative Example 7 | 2.5 : 1 | Zn | 2.21 | 0.79 | 3.01 | 52.00 |
| Comparative Example 8 | 2.5 : 1 | Mn | 2.27 | 0.85 | 3.13 | 51.84 |
| Comparative Example 9 | 2.5 : 1 | Sb | 4.29 | 1.51 | 5.80 | 49.37 |
| Comparative Example 10 | 2.5 : 1 | Ti | 1.10 | 0.54 | 1.63 | 53.83 |

As can be seen from the results in Table 6, when the mole ratio of 1,4-butanediol to polyethylene terephthalate is 2.5, the ratio of the total amount of EG and DEG is in the range of 1.63% to 5.80% by mole and that of BDO is in the range of 49.37% to 53.83% by mole in the polyester pellets of recycled polybutylene terephthalate produced in Comparative Example 6 without adding any catalyst or any one of Comparative Examples 7 to 10 of adding zinc, manganese or antimony catalyst or titanium catalyst without chelating ligands. However, the ratio of the total amount of EG and DEG is 0.47% by mole and that of BDO is 55.44% by mole in the polyester pellets of recycled polybutylene terephthalate produced in Example 2 of adding titanium catalyst with chelating ligands, in which the total amount of EG and DEG in the polyester pellets of recycled polybutylene terephthalate is reduced by 1.16% to 5.33% by mole and amount of BDO is increased by 1.61% to 6.07% by mole. In other word, when the mole ratio of 1,4-butanediol to polyethylene terephthalate is 2.5, the use of titanium catalyst with chelating ligands can effectively enhance the effect of 1,4-butanediol in substituting the ethylene glycol unit of polyethylene terephthalate (PET) and avoiding the formulation of diethylene glycol unit. Hence, the recycled polybutylene terephthalate produced by using titanium catalyst with chelating ligands has good hue and physical properties.

The foregoing description, for purposes of explanation, used specific nomenclature to provide a thorough understanding of the described embodiments. However, it will be apparent to one skilled in the art that many of the specific details are not required in order to practice the described embodiments. Thus, the foregoing descriptions of the specific embodiments described herein are presented for the purposes of illustration and description. They are not targeted to be exhaustive or to limit the embodiments to the precise forms disclosed. It will be apparent to one of ordinary skill in the art that many modifications and variations are possible in view of the above teachings.

The Summary and Abstract sections may set forth one or more but not all exemplary embodiments of the present invention as contemplated by the inventors, and thus, are not intended to limit the present invention and the appended claims in any way.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance. The breadth and scope of the present invention should not be limited by any of the above-described exemplary embodiments but should be defined only in accordance with the following claims and clauses and their equivalents.

The invention is defined in the non-limiting clauses:
1. A method for manufacturing a recycled polybutylene terephthalate, which comprises the following steps:
   1) in the presence of a titanium catalyst, mixing polyethylene terephthalate with 1,4-butanediol for depolymerization and transesterification to form a reaction mixture, wherein the titanium catalyst is chelated with a polybasic acid or a polyol, and the reaction mixture at least comprises tetrahydrofuran, water, ethylene glycol and 1,4-butanediol; and
   2) reducing the pressure to remove a part of the tetrahydrofuran and ethylene glycol from the reaction mixture followed by a condensation polymerization to produce the recycled polybutylene terephthalate.
2. The method according to clause 1, wherein the polybasic acid comprises a citric acid or a tartaric acid.
3. The method according to clause 1 or 2, wherein the polyol comprises a xylitol, a sorbitol, a maltitol, an erythritol or a mannitol.
4. The method according to any one of clauses 1 to 3, wherein the mole ratio of the 1,4-butanediol to the polyethylene terephthalate used in the step 1) is from 2:1 to 2.5:1.
5. The method according to any one of clauses 1 to 4, wherein in the reaction mixture, the amount of the ethylene glycol is at least 30% by weight relative to the total amount of the tetrahydrofuran, water, ethylene glycol and 1,4-butanediol.
6. The method according to any one of clauses 1 to 5, wherein the total amount of the tetrahydrofuran and water does not exceed 70% by weight relative to the total amount of the tetrahydrofuran, water, ethylene glycol and 1,4-butanediol.
7. The method according to any one of clauses 1 to 6, wherein the depolymerization and transesterification is carried out under a pressure of about 1 atm and a temperature of 210 to 230°C.
8. The method according to any one of clauses 1 to 7, wherein the condensation polymerization is carried out under a pressure of less than 0.1 atm and a temperature of 235 to 255°C.
9. A recycled polybutylene terephthalate manufactured by the method according to any one of clauses 1 to 8, which is a copolymer comprising a terephthalic acid unit, a isophthalic acid unit, and a butanediol unit.
10. The recycled polybutylene terephthalate according to clause 9, comprising an intrinsic viscosity (IV) between 0.5 and 1.0 dl/g and a melting point of at least 215°C.
11. The recycled polybutylene terephthalate according to clause 9 or 10, comprising a lightness (L) in color model coordinates of CIE L*a*b* equal to or greater than 74.0 and a b* value equal to or less than 4.0.
12. The recycled polybutylene terephthalate according to any one of clauses 9 to 11, which further comprises a diethylene glycol unit, wherein the amount of the diethylene glycol unit is less than 0.3% by weight relative to the total amount of the recycled polybutylene terephthalate.
13. The recycled polybutylene terephthalate according to any one of clauses 9 to 12, which further comprises an ethylene glycol unit, wherein the amount of the ethylene glycol unit is less than 0.1% by weight relative to the total amount of the recycled polybutylene terephthalate.
14. The recycled polybutylene terephthalate according to any one of clauses 9 to 13, which does not comprise an ethylene glycol unit.
15. The recycled polybutylene terephthalate according to any one of clauses 9 to 14, wherein the amount of the butanediol unit is more than 40.0% by weight relative to the total amount of the recycled polybutylene terephthalate.
16. A polyester fiber which comprises the recycled polybutylene terephthalate according to any one of clauses 9 to 15.
17. A polyester film which comprises the recycled polybutylene terephthalate according to any one of clauses 9 to 15.

## Claims

1. A method for manufacturing a recycled polybutylene terephthalate, which comprises the following steps:
1) in the presence of a titanium catalyst, mixing polyethylene terephthalate with 1,4-butanediol for depolymerization and transesterification to form a reaction mixture, wherein the titanium catalyst is chelated with a polybasic acid or a polyol, and the reaction mixture at least comprises tetrahydrofuran, water, ethylene glycol and 1,4-butanediol; and
2) reducing the pressure to remove a part of the tetrahydrofuran and ethylene glycol from the reaction mixture followed by a condensation polymerization to produce the recycled polybutylene terephthalate.

2. The method according to claim 1, wherein the polybasic acid comprises a citric acid or a tartaric acid.

3. The method according to claim 1 or 2, wherein the polyol comprises a xylitol, a sorbitol, a maltitol, an erythritol or a mannitol.

4. The method according to any one of claims 1 to 3, wherein the mole ratio of the 1,4-butanediol to the polyethylene terephthalate used in the step 1) is from 2: 1 to 2.5:1.

5. The method according to any one of claims 1 to 4, wherein in the reaction mixture, the amount of the ethylene glycol is at least 30% by weight relative to the total amount of the tetrahydrofuran, water, ethylene glycol and 1,4-butanediol.

6. The method according to any one of claims 1 to 5, wherein the total amount of the tetrahydrofuran and water does not exceed 70% by weight relative to the total amount of the tetrahydrofuran, water, ethylene glycol and 1,4-butanediol.

7. The method according to any one of claims 1 to 6, wherein the depolymerization and transesterification is carried out under a pressure of about 1 atm and a temperature of 210 to 230°C.

8. The method according to any one of claims 1 to 7, wherein the condensation polymerization is carried out under a pressure of less than 0.1 atm and a temperature of 235 to 255°C.

9. A recycled polybutylene terephthalate manufactured by the method according to any one of claims 1 to 8, which is a copolymer comprising a terephthalic acid unit, a isophthalic acid unit, and a butanediol unit.

10. The recycled polybutylene terephthalate according to claim 9, comprising an intrinsic viscosity (IV) between 0.5 and 1.0 dl/g and a melting point of at least 215°C, and/or comprising a lightness (L) in color model coordinates of CIE L*a*b* equal to or greater than 74.0 and a b* value equal to or less than 4.0.

11. The recycled polybutylene terephthalate according to claim 9 or 10, which further comprises a diethylene glycol unit, wherein the amount of the diethylene glycol unit is less than 0.3% by weight relative to the total amount of the recycled polybutylene terephthalate.

12. The recycled polybutylene terephthalate according to any one of claims 9 to 11, which further comprises an ethylene glycol unit, wherein the amount of the ethylene glycol unit is less than 0.1% by weight relative to the total amount of the recycled polybutylene terephthalate.

13. The recycled polybutylene terephthalate according to any one of claims 9 to 12, which does not comprise an ethylene glycol unit, and/or
wherein the amount of the butanediol unit is more than 40.0% by weight relative to the total amount of the recycled polybutylene terephthalate.

14. A polyester fiber which comprises the recycled polybutylene terephthalate according to any one of claims 9 to 13.

15. A polyester film which comprises the recycled polybutylene terephthalate according to any one of claims 9 to 13.
